# EUROPEAN PATENT APPLICATION

(11) **EP 3 933 035 A1**
(43) Date of publication of application: **05.01.2022**
(21) Application number: 20183914.9
(22) Date of filing: 03.07.2020
(51) Int. Cl.: C12N 5/0783, A61K 31/7084, A61K 35/12

(54) **COMPOSITIONS COMPRISING EXTRACELLULAR VESICLES AND STING STIMULATORY AGENTS**

(71) Applicant: Aarhus Universitet, 8000 Aarhus C (DK); Region Midtjylland, 8800 Viborg (DK)
(72) Inventor: Hansen, Aida Solhøj, 5932 Humble (DK); Deleuran, Bent Winding, 8230 Åbyhøj (DK); Jakobsen, Martin Roelsggaard, 8240 Risskov (DK); Krapp, Christian, 8400 Risskov (DK)
(74) Representative: Plougmann Vingtoft a/s

(57) **Abstract**

This present invention describes extracellular vesicles derived from activated T cells can prime macrophages for enhanced pro-inflammatory response towards stimulation with STING agonists. Further, a direct anti-tumoral effect of extracellular vesicles derived from activated T cells prior to intratumorally injection of cGAMP is demonstrated.

## Description

### Technical field of the invention

The present invention relates to compositions comprising extracellular vesicles derived from activated T cells and a STING stimulatory agent. In particular, the present invention relates to uses of such composition as medicaments, such as in the treatment or amelioration of cancers.

### Background of the invention

Anti-cancer immune responses within tumors are essential for controlling cancer development or elimination. Today, treatments that are foreseen to gear-up the immune system for improved anti-tumor immune responses are approved at high pace. Despite significant advances, only a minority of patients respond well to these new treatment options defined in broad terms as "immunotherapies".

Preclinical studies focusing on the innate immune system demonstrate that activation of the "Stimulator of interferon genes" (STING) pathway is essential for mounting an efficient anti-tumor response, mediated through T-cell activation and increased presentation of tumor antigens by dendritic cells (DCs). The involvement of tumor-infiltrating myeloid cells, such as macrophages and DCs, in activation and suppression of T-cell driven anti-tumor immunity is also well documented. Importantly, multiple recent preclinical and clinical data, shows that proper activation of the innate immune system in the tumor microenvironment (TME) are highly involved in orchestrating the level of adaptive anti-tumor immunity. Here, the innate immune pathway regulated by the two cellular factors cGAS and STING have achieved much attention. The cGAS-STING pathway plays a significant role in controlling tumor development due to sensing of cytosolic DNA, impaired DNA damage responses or chromosol instability. The canonical STING pathway involves multiple factors but most importantly, the enzyme cGAS detects accumulated cytosolic DNA, which results in the production of cyclic-GMP-AMP (cGAMP). cGAMP binds directly to STING and initiates STING activation; leading to downstream activation of IRF3 and NF-kB. These upregulate transcription of type I Interferon genes, cytokines and chemokines; supporting infiltration of immune cells that search and destroy cancer cells, and potentiates the cancer immunity cycle.

Interestingly, studies in murine cancer models have shown limited effect of immunotherapy in absence of STING [1-5], suggesting that early innate immune responses and endogenous STING activation within tumors is essential for anti-tumor effects. Furthermore, combining immunotherapy with STING agonist therapy increases the effect of tumor control [1,4-6]. Furthermore, indirect activation of the cGAS-STING pathway targeting other signaling molecules have also recently shown impressive effects for driving the TME from immunosuppressive to immunostimulant, and mounting an efficient anti-tumor T-cell response.

The presence of cytosolic DNA can result from infection of the cells with foreign bacteria or certain viruses, DNA damage responses and repair, micronucleus destructure, as well as from dying cells taken up by phagocytosis. Recently it was demonstrated that DNA capable of activating the cGAS-STING pathway can also be introduced into the cytosol by uptake of extracellular vesicles (EVs) carrying DNA on the surface [7,8].

EVs are nanometer-sized vesicles that contain several cellular components comprising proteins, lipids, DNA, and miRNA, and are produced by most cell types including immune cells and cancer cells amongst others. They are formed either by budding from the cell membrane as micro vesicles or formed inside the cells in endosomes and released by fusion of the multivesicular endosome with the cell membrane as exosomes. Micro vesicles have a size range between 100-1000 nm whereas exosomes have size range from 30-150 nm. They are released to the circulation and have a high stability and can travel long distances.

WO 2019/183578 A1 discloses compositions comprising EV, e.g., exosome, encapsulated STING agonists and methods of producing the compositions. WO 2019/183578 A1 is silent in respect of using extracellular vesicles derived from activated T cells.

It has been demonstrated that during antigen-presentation, the T cells release high numbers of extracellular vesicles that are transferred unidirectionally to antigen-presenting cells (APCs) and contain DNA capable of activating the STING signaling pathway in dendritic cells [7,9].

Hence, an improved method for preparing EVs would be advantageous, and in particular a more efficient and/or reliable cancer treatment protocol involving EVs would be advantageous.

### Summary of the invention

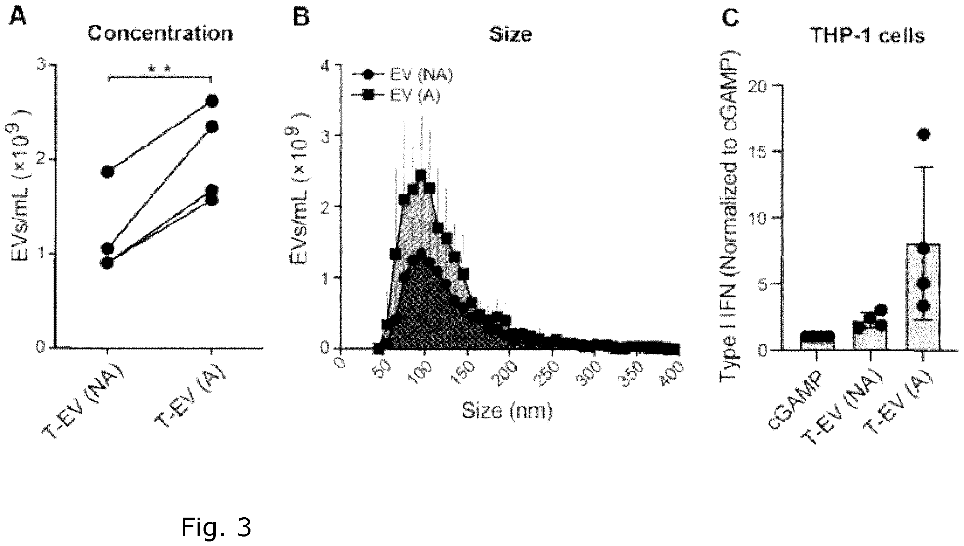

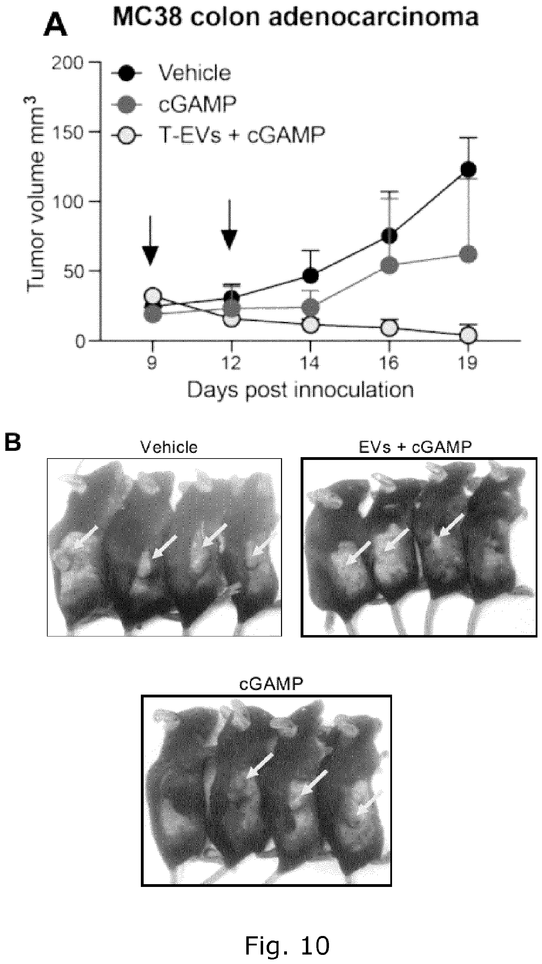

This invention describes that activated CD4⁺ T-cell derived EVs (extracellular vesicles derived from activated T cells, T-EVs) have unexpectedly the capability to prime e.g. myeloid cells, including but not limited to macrophages, for enhanced pro-inflammatory response towards stimulation with STING agonists, such as but not limited to the cyclic-di-nucleotide 2'3'-cGAMP (see example 2). Further, in Example 3 it is shown that T-EVs from activated CD4+ T cells sensitized the THP-1 cells for enhanced STING activation to a much larger extend that non-activated CD4+ T cells (Figure 3C). Thus, activation of the cells before EV isolation is important for obtaining sensitization of cells.
Importantly, it has also been identified that this effect is independent of cGAS supporting that the priming effect of cells is not mediated by sensing of intravesicular DNA (example 4). It is furthermore observed that murine CD4⁺ T-cell derived EVs stripped for surface associated DNA potentiate the effect of cGAMP in vivo. Finally, a direct anti-tumoral effect of activated EVs prior to intratumorally injection of cGAMP in the MC38 colon adenocarcinoma mouse model, leading to tumor remission is demonstrated (Example 9, figure 10A).

Thus, an object of the present invention relates to the provision of improved cancer treatment protocols using EVs.

Another object relates to the provision of improved cancer treatment protocol for combinatorial treatments.

Thus, one aspect of the invention relates to a process for producing isolated extracellular vesicles derived from activated T cells, the process comprising
a) activating a composition comprising purified T cells using anti-CD3 and/or anti-CD28 antibodies and/or IL-2; and
b) isolating extracellular vesicles derived from the activated T cells.

In another aspect, the invention relates to isolated extracellular vesicles obtained/obtainable by a process according to the present invention.

Yet another aspect of the present invention relates to a composition comprising extracellular vesicles derived from activated T cells and a STING stimulatory agent.

An additional aspect of the present invention is to provide a kit comprising
- extracellular vesicles derived from activated T cells; and
- a STING stimulatory agent.

A related aspect of the present invention relates to a combination of isolated extracellular vesicles derived from activated T cells and a STING stimulatory agent.

A further aspect the present invention relates to a composition, kit or combination according to the present invention for use as a medicament.

Yet a further aspect of the present invention relates to a composition, a kit or a combination according to the present invention for use in the treatment or amelioration of cancer.

An additional aspect relates to extracellular vesicles derived from activated T cells, for use in the treatment or amelioration of a subject suffering from cancer, wherein said subject has undergone cancer therapy or is undergoing cancer therapy, or who is scheduled for cancer therapy, with a different therapy, such as with chemotherapeutics or by radiation.

### Brief description of the figures

*Figure 1*
   T cell derived EVs sensitize the macrophages to STING activation. **A)** THP-1 cells were pre-treated with or without EV for 1 hr prior to stimulation with increasing amounts of cGAMP as indicated. After 20 hrs of stimulation the cell culture supernatant was analyzed for secretion of type I IFN and CXCL-10. Data shows mean +SEM of 2 technical duplicates and the data are representative of 2 independent experiments. **B)** and **C)** EVs from human CD4+ T cells were treated with DNase I prior to stimulation of B) THP-1 cells and C) human MDMs with or without cGAMP co-stimulation. The resulting type I IFN was determined after 20 hrs of stimulation. Data in B) shows mean +SD of 6 independent experiments and data in C) shows mean +SD of 6 different experiments. Each data point indicates the mean value of technical duplicates. * indicate p < 0.05, Wilcoxon test. **D)** THP-1 cells were pre-treated with or without EV for 1 hr prior to stimulation with either cGAMP (0.5µg/well), PolyI:C (0.1µg/well) or LPS (0.5µg/well). The resulting type I IFN was determined upon 20 hrs of stimulation. Data shows mean +SD of EV from 2 independent donors each in duplicate. * indicate p < 0.05, Wilcoxon test.
*Figure 2*
   T-cell derived EVs do not induce STING transcription. THP1 cells were stimulated with CD4+ T-cell derived EVs stripped for surface associated DNA. At the indicated time-points after stimulation the mRNA expression level of STING was determined. Data shows mean +/-SD of 2 technical duplicates and is representative of 2 independent experiments. UT = untreated.
*Figure 3*
   Synergistic effect is primarily induced by EVs from activated CD4+ T cells. EVs were isolated from CD4+ T cells that were either activated with plate-bound aCD3 and aCD28 or left non-activated for 48hrs. The EVs were analyzed by qNano for **A)** concentration and **B)** size distribution. Data in A) shows data from each of the 4 individual donors and B shows mean +SD of 4 independent donors. **C)** THP-1 cells were pre-treated with EVs from either non-activated or activated CD4+ T cells for 1 hr prior to stimulation with cGAMP (0.5µg/well). The resulting type I IFN was determined upon 20 hrs of stimulation. Data shows mean +/-SD from 4 independent experiments each performed in duplicate. The data is normalized to cGAMP. ** indicate p < 0.01, paired t-test. NA = non-activated. A = activated.
*Figure 4*
   The priming effect of EVs is STING-dependent but cGAS independent. **A)** Schematics of the cGAS-STING pathway. **B)** Wt, cGAS-/-, and STING-/- THP-1 cells were stimulated with CD4+ T-cell derived EVs stripped for surface associated DNA, for 1 hr prior to stimulation with cGAMP. The type I IFN response was determined after 20hrs of stimulation. Data shows mean +/-SD of 2 independent experiments. **C)** Western blot analysis of cell lysates from wt, cGAS-/-, and STING-/- THP-1 cells. Vinculin (VCL) was used as a loading control. Data represents 1 experiment.
*Figure 5*
   T-cell derived EVs prime THP-1 cells by inducing STING activation and NF-_{K}B phosphorylation dependent on TBK-1. THP-1 cells were stimulated with CD4+ T-cell derived EVs stripped for surface associated DNA for 1 hr prior to stimulation with cGAMP. At indicated time-points after cGAMP stimulation the supernatant and cells were harvested. **A)** The type I IFN response was determined at 1, 2, 4, and 6 hours after stimulation. Data shows mean +SD of 3 independent experiments. **B)** and **C)** Western blot analysis for corresponding cell lysates from A. Data are representative of 3 independent experiments. **D)** Wt, TBK1-/-, and STING-/- THP-1 cells were stimulated with CD4+ T-cell derived EVs or left untreated. At the indicated minutes (min.) after EV stimulation, the cells were harvested for Western blot analysis. Data are representative of 2 independent experiments. In all Western blot experiments Vinculin (VCL) was used as a loading control.
*Figure 6*
   EVs from activated CD4+ T cells induce phosphorylation of NF-_{K}B. Wt PMA differentiated THP-1 cells were stimulated with DNase I pre-treated EVs from either activated (A) or non-activated (NA) CD4+ T-cell. As controls the cells were either left unstimulated (UT) or stimulated with 0. 5µg 2'3'cGAMP- After 1hrs of EV stimulation, the cells were harvested for Western blot analysis. Data are representative of 1 experiment using EVs from different donors. Vinculin (VCL) was used as a loading control. NA = non-activated. A = activated.
*Figure 7*
   T-cell derived EVs alone induce secretion of proinflammatory cytokines in macrophages. Human monocyte derived macrophages (MDMs) were stimulated for 20 hrs with CD4+ T-cell derived EVs stripped for surface associated DNA. The resulting secretion of CXCL10 and IL-6 into the cell culture supernatant was determined by ELISA. Data shows mean +/- SD of 2 independent MDM donors each experiment done in technical duplicates. UT = untreated.
*Figure 8*
   T-cell derived EVs transfer proinflammatory cytokines to enhance macrophage function. **A)** EVs (3 x 10⁹) from activated human CD4+ T cells were analyzed for cytokine content by mesoscale. Data shows EVs from 2 different donors. **B)** THP-1 cells were stimulated with CD4+ T-cell derived EVs or recombinant cytokines; IL-4 (10pg/well), TNFa (1200pg/well), or IFNg (200pg/well) for 1 hr prior to stimulation with cGAMP. The type I IFN response was determined after 20hrs of stimulation. Data show mean + SEM of 2 technical replicates from 1 experiment.
*Figure 9*
   Murine CD4+ T cells derived EVs enhance STING activation and induce NF-_{K}B phosphorylation. Murine bone marrow derived macrophages (BMMs) were stimulated with EVs from murine CD4+ T cells with or without cGAMP co-stimulation. **A)** The secretion of IFN-b was assessed by ELISA after 20 hrs of stimulation. Data show mean +/-SD of 2 independent experiments each performed in technical duplicates. UT = untreated and received only lipofectamine. **B)** At indicated time-points after EV stimulation, the cells were harvested and analyzed by Western blotting for phosphorylation of NF-_{K}B. Data is from 1 experiment. Vinculin (VCL was used as a loading control.
*Figure 10*
   Intra tumoral treatment with EVs combined with cGAMP induce tumor regression of MC38. MC38 colon adenocarcinoma bearing mice (subcutaneous) were treated 2 times with 3 days interval (indicated by black arrows) with either Vehicle (PBS), murine CD4⁺ T cell derived EVs (1.5 x 10⁹) combined with cGAMP (10µg) or cGAMP alone (10µg) administered intratumorally. The tumor size was measured at the indicated days using the clipper method. **A)** Summarized tumor volume of the different groups indicated by mean +SD (n = 4 in each group). **B)** Pictures of each individual mouse receiving either vehicle, EVs + cGAMP or cGAMP alone at the termination of the experiment. Gray arrow indicates tumor or visible scar from previous tumor.
*Figure 11*
   Murine CD4+ T cells derived EVs induce type I IFN dependent on surface-associated DNA. Murine bone marrow derived macrophages (BMMs) were stimulated with murine T-EVs pre-treated with or without DNase I for 1hr to remove surface associated DNA prior to stimulation. The secretion of IFN-b was assessed by ELISA after 20 hrs of stimulation. Data show mean +/-SD of 2 technical replicates. UT = untreated and received only lipofectamine.

The present invention will now be described in more detail in the following.

### Detailed description of the invention

### Process for producing isolated immune-cell derived extracellular vesicles

Purified/isolated extracellular vesicles derived from activated T cells can be produced in different ways.
In an aspect the present invention relates to a process for producing isolated extracellular vesicles derived from activated T cells, the process comprising
a) activating a composition comprising purified T cells; and
b) isolating extracellular vesicles derived from the activated T cells.

In a related aspect the present invention relates to a process for producing isolated extracellular vesicles derived from activated T cells, the process comprising
a) activating a composition comprising purified T cells using anti-CD3 and/or anti-CD28 antibodies and/or IL-2; and
b) isolating extracellular vesicles derived from the activated T cells.

(Nano-sized) extracellular vesicles (EVs) can be secreted from cells by constitutive release after activation. The extracellular vesicles can be packaged with molecules specifically selected for a certain cell/tissue state and thereby communicate a molecular message of this cell/ tissue state to a surrounding and/or distant area. The EVs possess the natural machinery needed to selectively enter, and transmit, this complex molecular message efficiently into targeted cells.

Extracellular vesicle is a common term for a diverse class of vesicles shed from different cell types. Extracellular vesicles include exosomes and microvesicles.

Exosomes are small vesicles (such as around 30-150 nm in diameter) enclosed in multivesicular endosomes within the cell's cytoplasm. Exosomes are released from the cell after exocytosis of the multivesicular endosome. Microvesicles are 100-1000 nm in diameter and develop as buddings of the cell membrane.

In one embodiment, the extracellular vesicles involved in the processes, uses, compositions and kits provided herein may comprise both microvesicles and exosomes. In another embodiment, the extracellular vesicles are microvesicles or exosomes.

As shown in example 3 and figure 3, the isolated EV's has a size distribution mainly around 50-200 nm in diameter. Thus, in an embodiment the extracellular vesicles derived from activated T cells according to the invention (any aspect) have a diameter in the range 40-250 nm, such as in the range 50-200 nm, such as 50-150 nm, or such as in the range 80-120 nm.

Extracellular vesicles comprise a membrane surrounding the lumen of the extracellular vesicle. In addition, extracellular vesicles often comprise membrane bound molecules, such as polypeptides, lipids or DNA fragments. Likewise, the vesicular lumen can comprise a range of different molecules, including polypeptides, nucleic acids, small molecules, lipids, metabolites etc. The content of the lumen includes any structure, such as small molecules, which can be identified by enzymatic and/or chemical methods.

The processes, uses, compositions and kits provided herein generally relates to extracellular vesicles or part thereof, wherein the extracellular vesicles are derived from immune cells. Such extracellular vesicles are believed to contain agents, which are capable of modulating immune response.

The immune system is a host defense system comprising many biological structures and processes within an organism that protects against disease. To function properly, an immune system must detect a wide variety of agents, known as pathogens, from viruses to parasitic worms, and distinguish them from the organism's own healthy tissue. The immune system can be classified into subsystems, such as the innate immune system versus the adaptive immune system, or humoral immunity versus cell-mediated immunity.

The immune system relies on several different immune cells to protect the host system. These include T cells, NK cells, monocytes, macrophages, dendrites and/or B cells.

The extracellular vesicles of the present invention can in principle be derived from any immune cell. Examples of immune cells, from which extracellular vesicles referred to herein can be derived are T cells, NK cells, monocytes, macrophages, dendritic cells and/or activated B cells.

The extracellular vesicles of the present invention are preferably derived from T cells. T cells are a type of lymphocytes, which are a subtype of white blood cells. T cells can be distinguished from other lymphocytes, such as B cells and natural killer cells, by the presence of a T cell receptor on the cell surface. The cells play a central role in cell-mediated immunity and there are several subsets of T cells each having a distinct function.

Antigen-naive T cells expand and differentiate into memory and effector T cells. The memory T cells are long-lived and provide the immune system with "memory" against previously encountered pathogens. The effector T cells actively and immediately responds to a stimulus and aids in the fight against incoming pathogens. The effector T cells may comprise the following subtypes such as T helper cells, cytotoxic T cells and/or regulatory T cells.

Mature T-helper cells express the surface protein CD4 and are referred to as CD4⁺ T cells. CD4⁺ T cells recognize peptides presented on MHC class II molecules, which are found on antigen presenting cells (APCs). As a whole, they play a major role in instigating and shaping adaptive immune responses.

T cells can also be divided into alpha beta T cells and gamma delta T cells. The alpha beta T cells comprise alpha and beta chains on the cell receptor and are part of the adaptive immune system. The gamma delta T cells have invariant T-cell receptors, consisting of one γ(gamma) chain and one δ (delta) chain, with limited diversity. This T-cell receptor can effectively present antigens to other T cells. The gamma delta T cells are considered to be part of the innate immune system.

In a preferred embodiment, the immune cells are T cells and the extracellular vesicles or part thereof described herein are T-cell derived extracellular vesicles or part thereof. In one embodiment, the T cells are CD8⁺ T cells, naïve CD4⁺ T cells and/or naïve CD8⁺ T cells, and/or CD4⁺/CD8+ T cells. In one preferred embodiment the T cells are CD4⁺T cells, and even more preferably activated CD4⁺ cells.

In example 1, details for preparing the purified activated T-cells derived extracellular vesicles are also described. The process is also further described here. Extracellular vesicles derived from immune cells, such as T cells, can be generated both *in vivo* and *ex vivo.*

For *in vivo* production, the cells can be stimulated *in vivo* leading to release of extracellular vesicles from immune cells. These vesicles can then be extracted e.g. from a blood and/or tissue sample. The collected extracellular vesicles can then be purified and isolated from the samples.

For practical reasons, *ex vivo* production is however often the preferred method of generating extracellular vesicles. For *ex vivo* production, the relevant immune cells, such as T cells, are isolated, e.g. from blood samples and/or a tissue sample from e.g. the gut, the skin, the thymus, the spleen.

The term "activated cells" or "activated T cells" is a well-known term for the skilled person. The isolated immune cells, such as T cells, can be activated to release extracellular vesicles *in vitro* by stimulation. Immune cells can be activated by different methods available in the art for immune-cell activation. The activation can for example be done using antibodies, such as anti-CD3 and anti-CD28 antibodies or using phytohemagglutinin (PHA) and IL-2 in combination. In the example section, activation has been done using anti-CD3, anti-CD28 and IL-2 in combination. Thus, this combination is preferred.

The activation time used for the production of extracellular vesicles can differ and may result in the release of specific subpopulations of EVs. The activation time could be between 30 minutes to 5 days, 5 hours to 5 days, such as 24 hours to 5 days. In another embodiment, the activation of the cells can be repeated in several cycles of 3-5 days of stimulation followed by 10 days of resting and re-stimulation for additional 3-5 days. In a preferred embodiment, the activation time is 46 hours.

The extracellular vesicles can be isolated from *in vivo* produced blood and/or tissue samples and *in vitro* cultures of immune cells, such as T cells. There are several methods available in the art for isolation and purification of extracellular vesicles. For example, extracellular vesicles can be isolated by ultracentrifugation with or without a density gradient fractionation, size exclusion chromatography (SEC), immuno-selection, precipitation and/or filtration.

The extracellular vesicles used in the processes, uses, compositions and kits provided herein, may be used directly. However, in certain embodiments, the extracellular vesicles are subject to a modification treatment. For example, the extracellular vesicles can be stripped of surface bound DNA. Removal of surface bound DNA on the extracellular vesicles can be performed for example by treating the extracellular vesicles with a DNase enzyme. The resulting DNA free extracellular vesicles may be used in any of the processes, uses, compositions and kits disclosed herein.

The extracellular vesicles may also be loaded with other components relevant for their use. In one embodiment, stable DNA is loaded to the surface to the extracellular vesicles. Thus, in one embodiment, the extracellular vesicles are first stripped of the existing surface bound DNA followed by loading stable DNA to the surface.

In another embodiment, the extracellular vesicles are coated and/or loaded with one or more components selected from the group consisting of
- nucleic acids, such as cyclic-di-nucleotide (CDNs), such as cyclic 2'3' GMP-AMP (cGAMP), such as chemical stabilized forms of 2'3' cGAMP;
- enzymatic resistant double stranded DNA supporting intracellular cGAS activity;
- DNA damage repair (DDR) inhibitors including but not limited to: PARP inhibitors; ATM inhibitors, Topoisomerase inhibitors, DNA crosslinking agents, microtubule-targeting drugs, or antimetabolites;
- Small molecules capable of binding to and activating STING;
- Checkpoint inhibitors; and
- ENPP1 inhibitors.

Preferably, the EV's are coated and/or loaded with cyclic-di-nucleotide (CDNs).

Examples of stable DNA includes but not limited to, locked nucleic acid (LNA), Phosphorothioate (PS) bonds, phosphoramidite, Morpholine, 2'O-methyl; 2'Fluoro bases, inverted dT and ddT, which are resistant to degradation.

The modification treatment may also include loading the extracellular vesicles with different agents, which may be useful for the specific application of the extracellular vesicles.

Examples of the agents that can be loaded into the extracellular vesicles include peptides, proteins, protein fragments, lipids, metabolites, nucleotides and enzymatic and/or chemical identified structures of small molecules.

In one embodiment, the extracellular vesicles are loaded with one type of agent.

In another embodiment, the extracellular vesicles are loaded with multiple different agents, such as at least different 2 agents, such as at least different 3 agents, such as at least 4 different agents, such as at least 5 different agents, such as at least 10 different agents, such as at least 15 different agents, such as at least 25 different agents, such as at least 50 different agents.

The processes, uses, compositions, combinations and kits provided herein are particularly relevant for the treatment or amelioration of STING related disorders. STING is a protein, which is part of the innate immune response and involved in inducing an interferon and pro-inflammatory cytokine response during infection.

STING activation may be determined in a number of different ways including the following:
STING activation is induced by STING agonists, such as DNA. STING activation may be determined by determining STING phosphorylation. Said phosphorylation of STING may in particular be phosphorylation of Ser366 of STING.

Phosphorylation of STING, and particularly phosphorylation of Ser366 of STING may be determined in any useful manner. For example, STING activation may be determined as activation of expression of type I IFN or inflammatory cytokines in cells capable of expressing type I IFN or cytokines. Examples of such cells include macrophages, dendritic cells, keratinocytes, fibroblasts, monocytes, epithelia cells, B cells, or NK cells. Thus, STING activation may be determined by determining expression of type I IFN or cytokines in such cells.
Expression of type I IFN or cytokines may be determined by any useful manner, and methods are well-known in the art. For example, antibody-based methods are available and well-known.

STING activation may also be determined as activation of IFNβ promoter activity. Activity of the IFNβ promoter may for example be determined in recombinant cells comprising a nucleic acid construct encoding a reporter protein under the control of the IFNβ promoter. IFNβ promoter can also be determined in cell free expression systems allowing expression of a reporter protein under the control of the IFNβ promoter.

STING activation may also be determined as activation of NF-kB, STAT6, IRF3 and/or other signaling pathways. NF-kB, STAT6 and/or IRF3 activation may be determined by any useful manner. For example, NF-kB, STAT6 and/or IRF3 activation can be determined by phosphorylation of NF-kB, STAT6 and/or IRF3. Phosphorylation may be determined by any useful manner, and methods are well-known in the art. For example, antibody-based methods are available and well-known.

The processes, uses, compositions, combinations and kits provided herein generally relates to administering immune cell derived extracellular vesicles or part thereof together with a STING stimulatory agent. The STING stimulatory agent may enhance the effect of the extracellular vesicles.

STING may be activated by cytoplasmic DNA by a process involving several steps. The cytoplasmic DNA activates cyclic GMP-AMP synthase (cGAS) to produce cGAMP, which subsequently binds to and activates STING leading to expression of IFNs and other cytokines. Thus, cyclic dinucleotides can function as STING stimulatory agents thereby producing pharmacological activation of the innate immune response as well as priming of the adaptive immune response alone or in combination with other effector molecules and/or treatment options.

These stimulatory agents can include nucleic acids, cyclic-di-nucleotides, cGAS, cGAMP, and/or flavonoids.

In a further embodiment, the process of the invention further comprises the step c) of loading and/or coating the isolated extracellular vesicles, such as with DNA. In an embodiment, the DNA is protected DNA, such as DNAse protected DNA. Loading and coating may be performed by methods known in the art such as described in WO 2019/183578 A1.

In an additional embodiment, the isolated extracellular vesicles are free from DNA before loading/coating with external CDNs or external DNA (such as modified DNA, such as DNase protected DNA or similar).

In yet an embodiment, the DNA is modified DNA such as selected from the group consisting of locked nucleic acid (LNA), Phosphorothioate (PS) bonds, phosphoramidite, Morpholine, 2'O-methyl; 2'Fluoro bases, inverted dT and ddT.

In another preferred embodiment, in the step c) of loading and/or coating comprises loading or coating an agent selected from the group consisting of
- nucleic acids, such as cyclic-di-nucleotide (CDNs), such as cyclic 2'3' GMP-AMP (cGAMP), such as chemical stabilized forms of 2'3' cGAMP;
- enzymatic resistant double stranded DNA supporting intracellular cGAS activity;
- DNA damage repair (DDR) inhibitors including but not limited to: PARP inhibitors; ATM inhibitors, Topoisomerase inhibitors, DNA crosslinking agents, microtubule-targeting drugs, or antimetabolites;
- Small molecules capable of binding to and activating STING;
- Checkpoint inhibitors; and/or
- ENPP1 inhibitors.

The purified T-cells may be provided from different sources. Thus, in an embodiment, in step a) said purified T cells are from a subject in need of treatment or amelioration with the provided isolated immune-cell derived extracellular vesicles. Thus provided EVs may be used as an autograft, albeit allografts are also foreseen.

### Product by process

In another aspect, the invention relates to isolated extracellular vesicles obtained/obtainable by a process according to the present invention. Thus, another aspect relates to a composition according to the invention, wherein the isolated extracellular vesicles derived from activated T cells are obtained/obtainable by a process comprising
a) activating a composition comprising purified T cells using anti-CD3, anti-CD28 and IL-2; and
b) isolating activated extracellular vesicles derived from the activated T cells.

In a related aspect, present invention relates to a composition according to the invention, wherein the isolated extracellular vesicles derived from activated T cells are obtained/obtainable by a process comprising
a) activating a composition comprising purified T cells; and
b) isolating extracellular vesicles derived from the activated T cells.

### Composition

The extracellular vesicles derived from activated T cells disclosed in here may form part of a composition. Thus, a further aspect of the present invention relates to a composition comprising extracellular vesicles derived from activated T cells and a STING stimulatory agent. As also described in e.g. example 9, CD4⁺ T-cell derived EVs primes the tumor microenvironment to increase the responsiveness to cGAMP stimulation and result in the activation of a more potent immune response supporting remission of the tumor. Also in example 1 it is shown that T-EVs from activated CD4+ T cells sensitized the THP-1 cells for enhanced STING activation to a much larger extend that non-activated CD4+ T cells (Figure 3C).

It is to be understood that the term "derived from" could also mean "isolated from" or "obtained from".

In an embodiment, the STING stimulatory agent is an exogenous agent. In the present context, the term "exogenous agent" is to be understood as an agent (the STING stimulatory agent) not being derived from the (T-) cells from which the extracellular vesicles have be isolated (and therefore most come from an exogenous source).

In an embodiment, the STING stimulatory agent is not derived the T-cell from which the extracellular vesicles are derived, such as being exogenous DNA, such as DNAse protected DNA.

In an embodiment, the isolated extracellular vesicles derived from activated T cells comprise exogenously DNA, such as being exogenously delivered cyclic-di-nucleotides (CDNs) or such as DNAse protected DNA. As described in example 10, murine T-EVs contain surface associated DNA capable of inducing type I IFN. In the present context, the term "exogenously DNA" it is to be understood as DNA not derived from the (T-) cells from which the extracellular vesicles have be isolated.

In yet an embodiment, the isolated extracellular vesicles derived from activated T cells are obtained/obtainable by a process according to the present invention or is a composition according to the present invention.

In an embodiment, the composition is a pharmaceutical composition e.g. further comprising a diluent, carrier or excipient.

### Kit or combination

Since the composition of the invention comprises at least two components, an aspect of the invention relates to a kit comprising
- extracellular vesicles derived from activated T cells; and
- a STING stimulatory agent.

In an embodiment, the at least two components of the kit are in different containers, such as different vials.

A related aspect of the present invention relates to a combination of isolated extracellular vesicles derived from activated T cells and a STING stimulatory agent.

In an embodiment of the kit or combination, the extracellular vesicles, derived from activated T cells, are isolated extracellular vesicles according to the present invention.

As also outlined above, in an embodiment, the STING stimulatory agent is an exogenous agent.

In an embodiment, the STING stimulatory agent is selected from the group consisting of
- nucleic acids, such as cyclic-di-nucleotide (CDNs), such as cyclic 2'3' GMP-AMP (cGAMP), such as chemical stabilized forms of 2'3' cGAMP;
- enzymatic resistant double stranded DNA supporting intracellular cGAS activity;
- DNA damage repair (DDR) inhibitors including but not limited to: PARP inhibitors; ATM inhibitors, Topoisomerase inhibitors, DNA crosslinking agents, microtubule-targeting drugs, or antimetabolites;
- Small molecules capable of binding to and activating STING;
- Checkpoint inhibitors; and/or
- ENPP1 inhibitors.

### Medical use

As outlined above, the composition, kit and combination according to the present invention can be used as a medicament. Thus, yet an aspect the present invention relates to a composition, kit or combination according to the present invention for use as a medicament.

It is to be understood that the components of the kit or combination according to the invention may be administered simultaneously, successively in any order by any known therapeutically effective route.

In yet another aspect, the present invention relates to a composition, kit or combination according to the present invention for use in the treatment or amelioration of cancer. Again, example 9 and Fig. 10 show that CD4⁺ T-cell derived EVs prime the tumor microenvironment to increase the responsiveness to STING pathway engagement, directly through cGAMP stimulation, and result in the activation of a more potent immune response supporting remission of the tumor.

In an aspect, the invention relates to a composition, kit or combination according to the invention for use according to the invention, wherein the composition, kit or combination is for treatment or amelioration of a subject who has undergone therapy or is undergoing therapy, or who is scheduled for therapy of said cancer with a different therapy, such as with chemotherapeutics or by radiation. Phrased in another way, the composition, kit or combination is for use in combinatorial cancer treatment or amelioration.

In yet an aspect, the invention relates to extracellular vesicles (without an exogenous STING stimulatory agent), derived from activated T cells, for use in the treatment or amelioration of a subject suffering from cancer, wherein said subject has undergone cancer therapy or is undergoing cancer therapy, or who is scheduled for cancer therapy, with a different therapy, such as with chemotherapeutics (anti-cancer drugs) or radiation therapy. Phrased in another way, the extracellular vesicles derived from activated T cells (without an exogenous STING stimulatory agent), are for use in combinatorial cancer treatment or amelioration.

An effect of cancer therapy is cancer cell killing, which releases DNA (and other cellular components) to the nearby in vivo environment (and to the blood stream) and therefore act as the STING stimulatory agent. Thus, without being bound by theory, the extracellular vesicles derived from activated T cells (e.g. without a STING stimulatory agent), could be administered simultaneously with the cancer therapy or shortly after the cancer therapy, such as, but limited to, within 48 hours or within 24 hours from initiation of the cancer therapy session.

In an embodiment, the chemotherapeutic (anti-cancer drug) is selected from the group consisting of
- DNA damage repair (DDR) inhibitors including but not limited to: PARP inhibitors;
- ATM inhibitors, Topoisomerase inhibitors, DNA crosslinking agents, Antimetabolites, and microtubule-targeting drugs;
- Checkpoint inhibitors;
- Cytotoxic antibiotics;
- Alkylating agents;
- immunomodulating agents;
- Small molecule drugs; and
- antibodies that stimulate the immune response to a given cancer.

The skilled person will know of other types of chemotherapeutics and also specific drugs. Thus, the above list is a non-limiting list of chemotherapeutics according to the present invention, which may cause release of cellular components, which may then function as STING stimulatory agents in vivo.

Similar, the skilled person will know of different types of radiation therapies.

In an embodiment, the cancer is selected from the group consisting of melanoma, breast cancer, colon cancer, prostate cancer, liver cancer, lung cancer, glioblastoma, head and neck cancer and/or lymphoma.

In another embodiment the cancer is selected from the group consisting of breast
cancer, head and neck cancer, uterine cancer, brain cancer, skin cancer, renal cancer, lung cancer, colorectal cancer, prostate cancer, liver cancer, bladder cancer, kidney cancer, peritoneal cancer, pancreatic cancer, thyroid cancer, esophageal cancer, eye cancer, stomach (gastric) cancer, gastrointestinal cancer, carcinoma, sarcoma, leukemia, lymphoma, myeloma, or a combination thereof.

In a further embodiment, the cancer is a metastatic cancer, a refractory cancer (e.g., a cancer refractory to previous cancer therapy), and/or recurrent cancers.

In another embodiment, the cancer is associated with STING activity, such as insufficient STING activity.

In yet another embodiment, the composition kit or combination is administered intravenous (IV), intratumoral (IT), or subcutaneous (SC).

### In vitro use

The compositions, kits and combinations according to the invention may also find use in vitro. Thus, an aspect of the present invention relates to an in vitro method of stimulating STING activity in a cell, said method comprising contacting said cell with a composition, a kit or a combination according to the present invention.

In another aspect, the invention relates to the use of a composition, kit or combination according to the invention, for in vitro stimulation of STING activity in a cell.

### Additional aspects

In an additional aspect, the invention relates to a method of preventing, treating and/or ameliorating a cancer in a subject in need thereof, said method comprising administering isolated extracellular vesicles derived from activated T cells together with a STING stimulatory agent to the subject in need thereof.

In an embodiment, the isolated extracellular vesicles derived from activated T cells are obtained by a process according to the invention.

In yet an additional aspect, the invention relates to a method of preventing, treating and/or ameliorating a cancer in a subject in need thereof, wherein said subject has undergone cancer therapy or is undergoing cancer therapy, or who is scheduled for cancer therapy, with a different therapy (than the isolated extracellular vesicles derived from activated T cells), such as with chemotherapeutics or by radiation, said method comprising administering isolated extracellular vesicles derived from activated T cells to the subject in need thereof. Again, phrased in another way, the extracellular vesicles derived from activated T cells (without an exogenous STING stimulatory agent), may be for use in combinatorial cancer treatment or amelioration.

It should be noted that embodiments and features described in the context of one of the aspects of the present invention also apply to the other aspects of the invention.

All patent and non-patent references cited in the present application, are hereby incorporated by reference in their entirety.

The invention will now be described in further details in the following non-limiting examples.

### Examples

### Example 1 - Materials and methods

### Isolation of human CD4⁺ T cells

Human peripheral blood mononuclear cells (PBMCs) were isolated from buffy coats from healthy donors using Ficoll-Paque PLUS (GE Healthcare Bioscience). The PBMCs were cryopreserved in 40% heat-inactivated fetal bovine serum (FCS, Gibco), 50% RPMI-1640 (Sigma-Aldrich) and 10% DMSO (Sigma-Aldrich) and stored at -150 °C. CD4⁺ T cells were isolated from PBMCs by negative selection using EasySep Human CD4⁺ T Cell Isolation Kits (Stemcell) according to manufacturer's instructions.

### Isolation of murine CD4⁺ T cells

The spleen was dissected from healthy C57BL/6 mice and a single cell suspension of splenocytes was prepared by pressing the spleens gently through a 70µm nylon cell strainer. CD4⁺ T cells were isolated from the splenocytes by negative selection using EasySep Mouse CD4⁺ T Cell Isolation Kits (Stemcell) according to manufacturer's instructions.

### Generation of extracellular vesicles (EVs)

For generation of EVs the cells were cultured in medium containing EV-free FCS prepared by ultracentrifugation at 100,000 × g for 20 hours to remove all EVs from the serum.

CD4⁺ T cells were activated *in vitro* resulting in release of high numbers of extracellular vesicles (EVs) into the cell culture supernatant. For activation of human CD4⁺ T cells, approximately 4-6 × 10⁶ isolated human CD4⁺ T cells/well were stimulated in a 6-well plate pre-coated with 1µg/ml aCD3 (clone UCHT-1, R & D System) and cultured in RPMI-1640 supplemented with 10% EV-free FCS, 10 mM HEPES (Gibco), 2mM glutaMAX (Gibco), 200 IU/ml Penicillin, 100 µg/ml Streptomycin, 1 µg/ml soluble aCD28 (clone CD28.2, BD Pharmingen) and 10 ng/ml recombinant human IL-2 (Roche). For activation of murine CD4⁺ T cells, approximately 4-6 × 10⁶ isolated murine CD4⁺ T cells/well were stimulated in a 6-well plate pre-coated with 1µg/ml aCD3 (clone 145-2C11, BD Pharmingen) and 1µg/ml and aCD28 (clone 37.51, BD Pharmingen) and cultured in RPMI-1640 supplemented with 10% EV-free FCS, 10 mM HEPES (Gibco), 2mM glutaMAX (Gibco), 1X non-essential amino acids (Gibco) 200 IU/ml Penicillin, 100 µg/ml Streptomycin, 50µM beta-mercaptoethanol and 10 ng/ml recombinant human IL-2 (Roche). Both human and murine CD4⁺ T cells were cultured for 46 hrs under humidified conditions at 37°C, 5% CO₂.

### Isolation of EVs

EVs were isolated from the cell culture supernatant by differential centrifugation. In brief, the cell culture supernatant was centrifuged for 10 minutes at 1000 × *g* followed by 20 minutes at 2000 × *g* and 35 minutes at 10,000 × *g*. The EVs were then pelleted from the supernatant by centrifugation for 2 hrs at 100,000 × *g.* All centrifugation steps were performed at 4°C and the ultracentrifugation was done using a TST 41.14 rotor (Sorval). The EV pellet was resuspended in either PBS (Sigma-Aldrich), T cell media or physiological water (Sigma-Aldrich). To remove surface associated DNA the EVs were incubated with 10 units DNase I (Thermo Scientific) for 1 hour at room temperature.

### Nanoparticle tracking analysis

Nanoparticle tracking analysis was performed on NanoSight LM10 (Malvern Instruments) with a 405 nm laser and was used to determine the concentration of EVs used for all stimulation experiments. EVs were diluted 100 times in PBS and measurements were performed in three times of 60s video captures of each sample with camera level 15 and detection threshold 10 for all analysis. The data were analyzed using software version 3.1.

### EV characterization

A qNano Gold (Izon Bioscience) equipped with a NP100 Nanopore (analysis range 50-330 nm, Izon Bioscience) for tunable resistive pulse sensing (TRPS) was used to determine size and concentration of the EVs. The EV samples were diluted in PBS and the EV samples were analyzed under identical settings - the same diluent, stretch (47mm), pressure (10Pa) and voltage (0.6mV) as used for CPC100 calibration particles (Izon Bioscience). The EV concentration and size distribution were determined using the "Izon control suite" software (Izon Bioscience).

### Mass Spectrometry

For mass spectrometry (MS) analysis, EVs from 24-30 × 10⁶ CD4⁺ T cells either activated or left non-activated for 48 hrs, were isolated as described above. The EV-pellets were washed in PBS and centrifuged for 1 hour at 100,000 × *g*. The EVs were resuspended in 50 µl PBS and digested according to the Filter Aided Sample Preparation (FASP) method. In short, the samples were loaded onto a 10 kMW spin filter and washed in 6M Urea, DTT and Iodoacetamide solution. The samples were digested with trypsin overnight at 37°C. The filters were centrifuged at 14,000 × *g* for 30min at 20°C and the eluate were collected and acidified with 5% formic acid. The tryptic peptides were micro purified using Empore SPE Disks of C18 octadecyl packed in 10 µL pipet tips. Nano-electrospray ionization tandem mass spectrometry (MS/MS) analyses were performed on a NanoLC 415 (Eksigent) connected to a TripleTOF 6600 mass spectrometer (AB Sciex). The micro purified peptides were dissolved in 0.1% formic acid, injected, and trapped on an in-house packed trap column (2 cm × 100 µm inner diameter) with RP ReproSil-Pur C18-AQ 3-µm resin (Dr. Maisch HPLC GmbH). Peptides were eluted from the trap column and separated on a 15-cm analytical column (75 µm inner diameter) packed in-house in a pulled emitter with RP ReproSil-Pur C18-AQ 3-µm resin (Dr. Maisch HPLC GmbH). Elution from the analytical column was performed with a linear gradient from 5 to 35% phase B (90% acetonitrile with 0.1% formic acid) over 20 or 50 min. The collected MS files were converted to Mascot generic format (MGF) using the AB Sciex MS Data Converter beta 1.1 (AB Sciex) and the "proteinpilot MGF" parameters. The generated peak lists were searched against the Swiss-Prot database using an in-house Mascot search engine (Matrix Science). Search parameters: Trypsin 1 miscleavage, carbamidomethyl (iodoacetamide) was set as a fixed modification with peptide tolerance and MS/MS tolerance set to 10 ppm and 0.1 Da, respectively.

### Mass Spectrometry for cGAMP analysis

For analysis of cGAMP presence in EVs, 6-9.5 × 10⁹ EVs from activated human CD4+ T cells were resuspended in 1ml v/v 80%, 2% acetic acid. The samples were incubated for 15min at RT and added to NH₂ SPE-columns pre-conditioned with first methanol and water. Columns containing the EV samples were washed with methanol and subsequently water and cGAMP was eluted with alkaline methanol (v/v 80% methanol, 5% NH₄OH). The eluted samples were dried using a vacuum centrifuge and dissolved in 50µl 0.1% formic acid. 10µl of the samples were injected on a HSS T3 (2.1 × 100mm) LC-column. cGAMP was LC-ESI-MS/MS using the following ion phases: *m*/*z* 338 > 152, 338 > 524, 338 > 136, 336 > 134, and 336 > 150. To obtain semi quantitative results, the samples were spiked with 0, 50, 100 and 200 nM cGAMP respectively. The spikes samples were purified and used as extern calibrators. ESI(+) production was used for quantification.

### Cell culture

All cells were cultured under humidified conditions at 37°C and 5% CO₂.
Human acute monocytic leukemia cell line (THP-1) was cultured in RPMI-1640 (Sigma-Aldrich) supplemented with 10% heat-inactivated fetal calf serum (FCS, Gibco), 200 IU/ml Penicillin, 100 µg/ml Streptomycin and 600 µg/ml glutamine (hereafter termed RPMI complete). Infection with mycoplasma was tested and ruled out on a monthly basis using the Lonza MycoAlert kit (LT07-703). THP-1 cells were differentiated into phenotypically adherent macrophages by stimulation with 100 nM Phorbol 12-myristate 13-acetate (PMA, Sigma-Aldrich) in RPMI complete for 24 hours. The medium was then refreshed with normal RPMI complete allowing the cells to further differentiate an additional day and they were hereafter defined as macrophages. THP-1 clones harboring knock-out mutations in genes encoding STING and cGAS was cultured and activated similar to the wt THP-1 cells.

Human monocyte-derived macrophages (MDMs) were differentiated from PBMCs by culturing the cells in RPMI complete medium supplemented with 10% AB-positive human serum and 15 ng/ml M-CSF (PeproTech, 300-25-100UG). After 2 days of culturing, the medium was changed to Dulbecco's Modified Eagle Medium (DMEM, Sigma-Aldrich) supplemented with 200 IU/ml Penicillin, 100 µg/ml Streptomycin, 600 µg/ml glutamine, 10% AB-positive human serum and 15 ng/ml M-CSF and the cells were allowed to further differentiate for additional 6-8 days.

Murine bone marrow was extracted from femur and tibia from 8-12 weeks old healthy C57B6/J mice by centrifugation and cryopreserved at -150°C in 40% heat-inactivated fetal bovine serum (FCS, Gibco), 50% RPMI-1640 (Sigma-Aldrich) and 10% DMSO (Sigma-Aldrich). Bone marrow derived macrophages (BMMs) were differentiated by culturing bone marrow cells in presence of L929 SN containing the necessary M-CSF for differentiation. L929 SN was prepared by collecting the supernatant (SN) from confluent L929 cells. For differentiation, bone marrow cells were cultured in RPMI-1640 complete medium supplemented with 20% L929 SN. After 2 days the cells were refreshed with RPMI complete medium supplemented with 40% L929 SN and on day 4 the medium was changed to RPMI complete supplemented with 20% L929 SN. After 6 days the BMMs were fully differentiated and harvested using Accutase and seeded for further stimulation.

### Stimulation of cells in vitro

For stimulation of the cells *in vitro* were used 5 × 10⁴ cells/well in a 96-well plate of either PMA-differentiated THP-1 cells, MDMs or BMMs. The cells were seeded 1 day prior to stimulation allowing the cells to adhere to the bottom of the culture well. Upon stimulation, the culture medium was removed from the cells and 50 µl EV-free medium containing 1-6 × 10⁹ EVs, or 50 µl EV-free medium alone was added to the cells and they were incubated at 37°C for 1 hr. 2'3'-cGAMP (InvivoGen) (0.5 µg/well) or poly:IC (0.1µg/well) were mixed with Lipofectamine-2000 (Invitrogen) in a ratio of 1:1 according to manufacturer's instructions. This mix was diluted with EV-free media and added to the cells in a volume of 100µl. The cells were incubated at 37°C for 20-24 hours if not otherwise indicated in the figures.

### Real-time PCR

RNA was extracted from the cells using the RNeasy mini kit (Qiagen) according to manufacturer's instructions. The concentration of the isolated RNA was determined using Nanodrop and 250ng RNA was converted into cDNA using the iScript cDNA synthesis kit (Bio-Rad) according to the manufacturer's instruction. The cDNA was diluted by a final factor 25 for the real-time PCR analysis. Real-time PCR analysis of STING and ACTB was performed using the TaqMan Fast advanced Mastermix (Thermo Fisher Scientific) and the following TaqMan gene expression assays: Hs00736955_g1 (STING/TMEM173) and Hs01060665_g1 (ACTB). The analysis was done using the AriaMX Real-time PCR System (Agilent Technology).

### Functional type I IFN

Bioactive functional type I IFN was quantified in supernatants using the reporter cell line HEK-Blue IFN-α/β (Invivogen) according to manufacturer's instructions. This cell line expresses secreted alkaline phosphatase (SEAP) under control of the IFN-α/β inducible ISG54 promoter. The cell line was maintained in DMEM supplemented with GlutaMax-I (Gibco, Life Technologies), 10% heat-inactivated FCS, 100 µg/mL streptomycin and 200 U/mL penicillin, 100 µg/mL normocin (InvivoGen), 30 µg/mL blasticidin (InvivoGen) and 100 µg/mL zeocin (InvivoGen). For measurement of functional type IFN, cells were seeded at 3 × 10⁴ cells/well in 96-well plates in 150 µL medium devoid of Blasticidin and Zeocin. The following day 50 µl of the supernatant for analysis were added to the HEK-Blue cells. SEAP activity was assessed by measuring optical density (OD) at 620 nm on a microplate reader (ELx808, BioTEK). The concentration was determined from a standard curve made with IFN-α (IFNa2 PBL Assay Science) ranging from 2 to 500 U/ml.

### Enzyme-linked Immunosorbent assay (ELISA)

Protein levels of the cytokines CXCL-10 and IL-6 in the cell culture supernatants were determined using the following ELISA kits: CXCL-10 and IL-6 (both from R&D System) according to manufacturer's instructions.

### Mesoscale V-Plex

3 × 10⁹ EVs from activated human CD4+ T cells were lyzed using ml v/v 2.5% Triton-X-100, 0.05% Tween-20. This lysate was analyzed for presence of IFNγ, IL-1β, IL-2, IL-4, IL-6, IL-8, IL-10, IL-12p70, IL-13 and TNFα using V-plex (Meso Scale Discovery^{®}, V-PLEX^{®} Proinflammatory Panel 1 (human)) according to manufacturer's protocol. Samples were diluted 1:2.

### Western blotting

Cellular protein expression was analyzed by Western blotting. Cell lysis buffer was made by mixing Pierce RIPA buffer (Thermo Scientific) supplemented with 10 mM NaF, 2X complete protease cocktail inhibitor (Roche), 2X protease and phosphatase inhibitor (Pierce) in a 1:1 dilution with Laemmli Lysis Buffer (Sigma-Aldrich). Benzonase (Sigma-Aldrich) were added to the lysis buffer in a volume of 1µl/ml lysis buffer. In general, 60µl of this lysis buffer were added to a single well in a 96-well plate and the. The lysate was denatured at 95 °C for 5 minutes and 15 µl lysate was separated on a Criterion Precast Gel, 4-12% Tris-HCI (Bio-Rad) with XT MOPS running buffer (Bio-Rad). The separated proteins were transferred to a Trans-Blot Turbo 0.2 µm PVDF membrane using the Trans-Blot Turbo Transfer System (Bio-Rad). Membranes were washed in TBS supplemented with 0.05% Tween-20 (TBS-T) and blocked in 5% fresh made skim-milk (Sigma-Aldrich) in TBS-T. Following antibodies were diluted in 1-5% BSA (Roche): anti-cGAS (Cell Signaling, D1D3G), anti-STING (Cell Signaling, D2P2F) and anti-vinculin (Sigma Aldrich, v9131), anti-phospho-STING (Cell Signaling, D7C3S, anti-phospho-TBK1 (Cell Signaling, D52C2), anti-TBK1 (Cell Signaling), anti-phospho-IRF3 (Cell Signaling, D601M). The membranes were incubated with primary antibodies overnight at 4°C. The following secondary antibodies were diluted in 1% skim-milk: peroxidase-conjugated F(ab')2 donkey anti-mouse IgG (H+L), peroxidase conjugated affinipure F(ab')2 donkey anti-rabbit IgG (H+L), and peroxidase conjugated F(ab')2 donkey anti-goat IgG (H+L) (all purchased from Jackson Immuno Research). The membranes were incubated with secondary antibodies for 1 hour at room temperature. The membranes were exposed using Clarity Western ECL Blotting Substrate.

### Mice

C57BL/6 mice from Janvier were housed in the animal facility in the Bartholin Building at Aarhus University, under conditions according to the recommendations of The Animal Experiments Inspectorate under the Ministry of Environment and Food of Denmark. The Study was conducted in accordance with The Animal Ethics Council. For all experiments were used female mice with an age of 7-9 weeks at initiation of the experiments.

### Generation of tumor xenografts

MC38 (Kerafast) colon adenocarcinoma were cultured in DMEM (Sigma Aldrich) supplemented with 10% FCS, 2mM Glutamine, 1000 U/ml penicillin and 1000 µg/ml streptomycin. The cells were cultured under humidified conditions at 37°C and 5% CO₂. The cells were split before they reached 100% confluency and were detached from the cell culture flask using 0.25 % trypsin (Gibco) in PBS with 0.02% EDTA (Invitrogen). To establish a tumor in the mice, 1 × 10⁶ MC38 cells in 50µl were injected subcutaneously into the right flank of the mice under isoflurane anesthesia.

### In vivo treatment

The *in vivo* experiment was conducted when the tumors reached a size of 20-40 mm³. The mice were grouped randomly and treated intratumorally (IT) 2 times with 3 days interval, with 30µl containing either 10µg 2'3'-cGAMP Vaccigrade (Invivogen), 1.5 × 10⁸ EVs diluted in physiological water (Sigma-Aldrich) or a combination. The tumor size was measured regularly using a caliper and the tumor volume was calculated using the formula: Tumor Volume (mm³) = L^{∗}W^{∗}H^{∗}3.14/6 where, L = Length (mm); W = Width (mm); H = Height (mm). At the end of the experiment or when the tumor reached a size of 1000 mm³ the mice were euthanized.

### Example 2 - T cell derived EVs sensitize macrophages to STING activation

### Aim of study

Assess the role of CD4⁺ T-cell derived extracellular vesicles (T-EVs) for the function of macrophages towards STING activation.

### Materials and methods

See example 1.

### Results

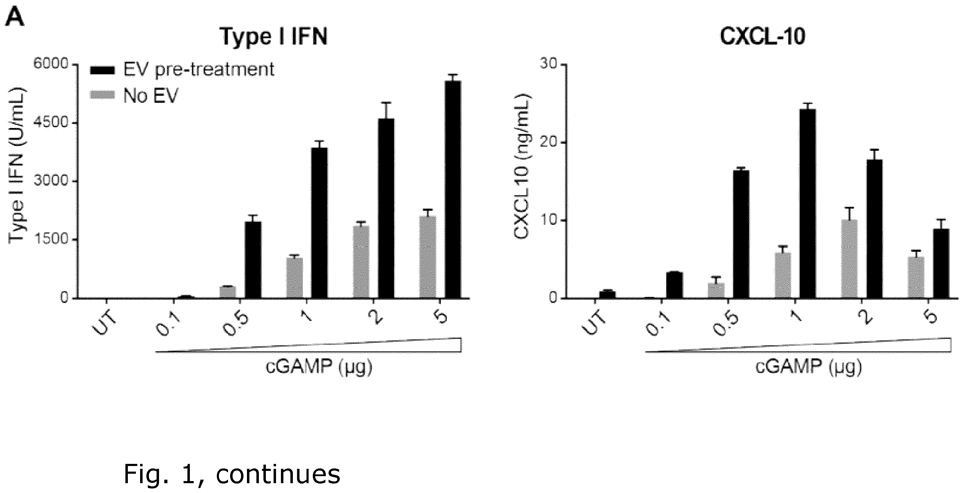

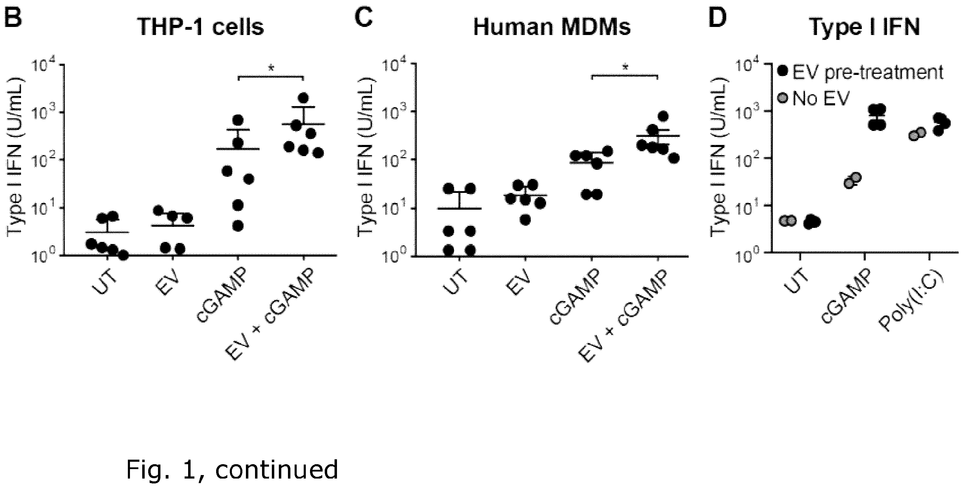

In order to assess the role of CD4⁺ T-cell derived extracellular vesicles (T-EVs) for the function of macrophages towards STING activation, we pre-treated PMA-differentiated THP-1 cells with T-EVs and stimulated the cells with increasing amounts of the natural STING ligand 2'3'-cGAMP. This resulted in enhanced secretion of Type I IFN and CXCL-10 regardless of the amount of cGAMP added to the cells and importantly made the THP-1 cells respond robustly to as low as 0. 5µg cGAMP (Fig. 1A and 1B). This was not restricted to THP-1 cells, as also primary human monocyte-derived macrophages (MDMs) responded robustly to low dosage of cGAMP when pre-treated with T-EVs (Fig. 1C). We further examined whether T-EVs also modulated the response of macrophages to stimulation of another pattern recognition pathway induced by Poly:IC and found that T-EVs did not enhance the response to Poly:IC (Fig. 1D). This indicates that T-EVs sensitize macrophages to respond to danger signals related to the STING pathway activation. Since it was demonstrated previously that EVs in certain circumstances may carry surface-associated DNA we used only T-EVs that were pre-treated with DNase I prior to stimulation of macrophages.

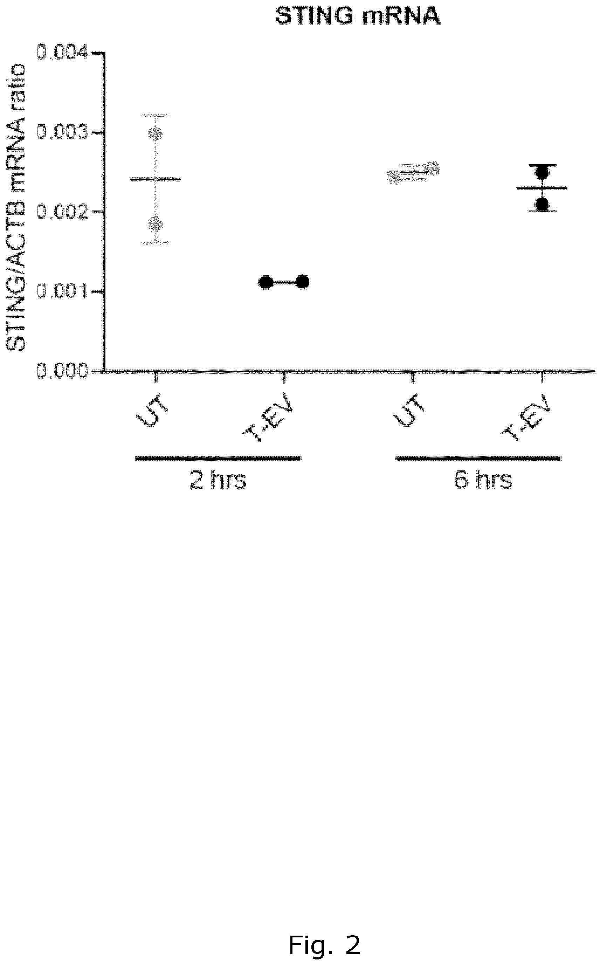

To examine whether the enhanced STING activation resulted from T-EVs directly inducing transcription of STING, we analyzed the expression level of STING mRNA following 2 and 6 hrs of T-EV stimulation and found no difference in the mRNA level of STING between T-EV and untreated THP-1 cells (Fig. 2).

### Conclusion

In sum, this indicates that T-EVs sensitize macrophages to respond to danger signals related to the STING pathway activation.

### Example 3 - Synergistic effect is primarily induced by EVs from activated CD4⁺ T cells

### Aim of study

To investigate whether the cellular sensitization was induced by EVs from CD4⁺ T cells in general or specifically induced by EVs from activated CD4⁺ T cells.

### Materials and methods

See example 1.

### Results

We isolated T-EVs from CD4⁺ T cells that were either stimulated with anti-CD3/anti-CD28 or left unstimulated and analyzed the concentration and size distribution of the secreted EVs in the cell culture supernatant. Activation of CD4⁺ T cells increased the release of T-EVs into the cell culture supernatant (Fig. 3A) but there was no difference in the size distribution between T-EVs from activated and non-activated CD4⁺ T cells (Fig. 3B). Interestingly, when added to a culture of PMA-differentiated THP-1 cells we observed that primarily T-EVs from activated CD4⁺ T cells sensitized the THP-1 cells for enhanced STING activation (Fig. 3C).

### Conclusion

The data indicates that the T-EVs from activated CD4⁺ T cells might carry specific content capable of sensitizing the STING signalling pathway in macrophages. We did a mass spectrometry analysis of the global protein content in T-EVs from both activated and non-activated CD4⁺ T cells. We identified 299 peptides comprising several T-cell surface glycoproteins including CD2, CD3, CD4, CD5, and CD44. There was a lot of variation in the peptides identified between different donors and we did not observe any clear differences in the proteomic profile between T-EVs from activated and non-activated T cells (Data not shown).

### Example 4 - The synergistic effect of T-EVs is dependent of STING and independent of cGAS

### Aim of study

To address whether the synergistic effect of the T-EVs could be caused by DNA present inside T-EVs (protected from the DNase I treatment).

### Materials and methods

See example 1.

### Results

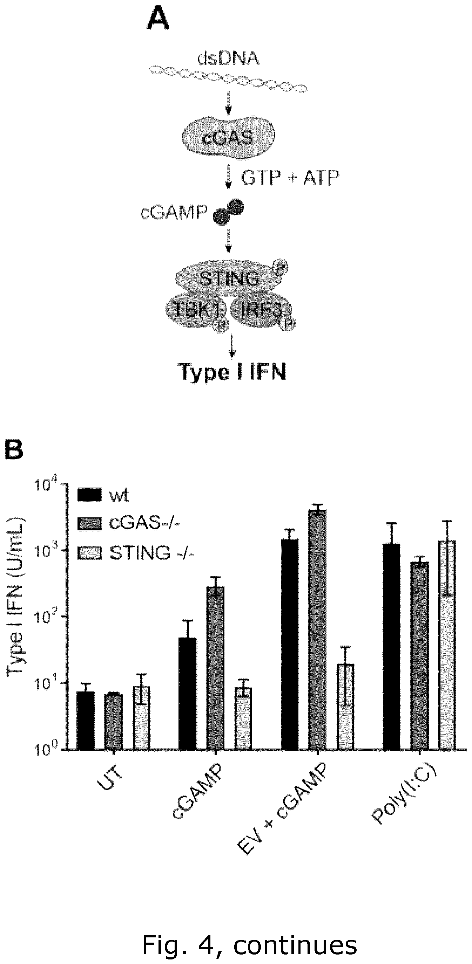

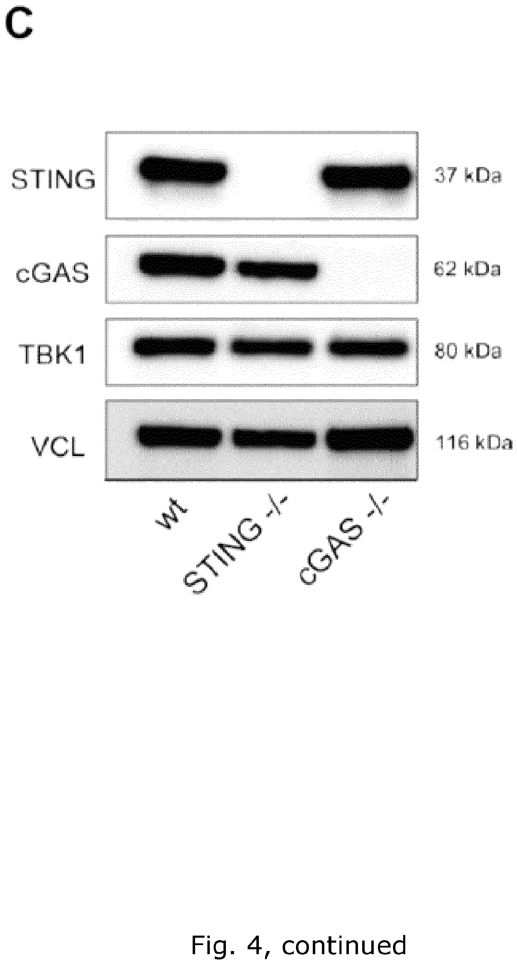

CRISPR-Cas9 cGAS-deficient THP-1 cells were pre-treated with T-EVs prior to cGAMP stimulation. The cGAS knock-out (KO) THP-1 cells responded to T-EV priming similar to WT THP-1 cells (Fig. 4A-C). These data support that the synergistic effect of T-EVs is not mediated by intra-vesicular DNA.

In parallel, we pre-treated CRISPR-Cas9 STING-deficient THP-1 cells with T-EVs and found that STING KO THP-1 cells did not respond to either cGAMP alone or after T-EV pre stimulation demonstrating that the MoA by T-EVs are dependent on STING. It has been suggested that cGAMP can be transported between cells in EVs. Therefore, we finally did a mass spectrometry-based detection assay of cGAMP in the T-EVs. However, T-EVs did not contain detectable levels of cGAMP (Data not shown).

### Conclusion

Together these data demonstrate that the synergistic effect of T-EVs on enhancing STING activation is not mediated by neither DNA nor cGAMP inside the T-EVs.

### Example 5 - T-EVs prime THP-1 cells by inducing NF-kB activation dependent on TBK-1

### Aim of study

To investigate by a time-kinetic experiment whether the enhanced type IFN response following T-EV and cGAMP co-stimulation was a result of early priming of the THP-1 cells by the T-EVs.

### Materials and methods

See example 1.

### Results

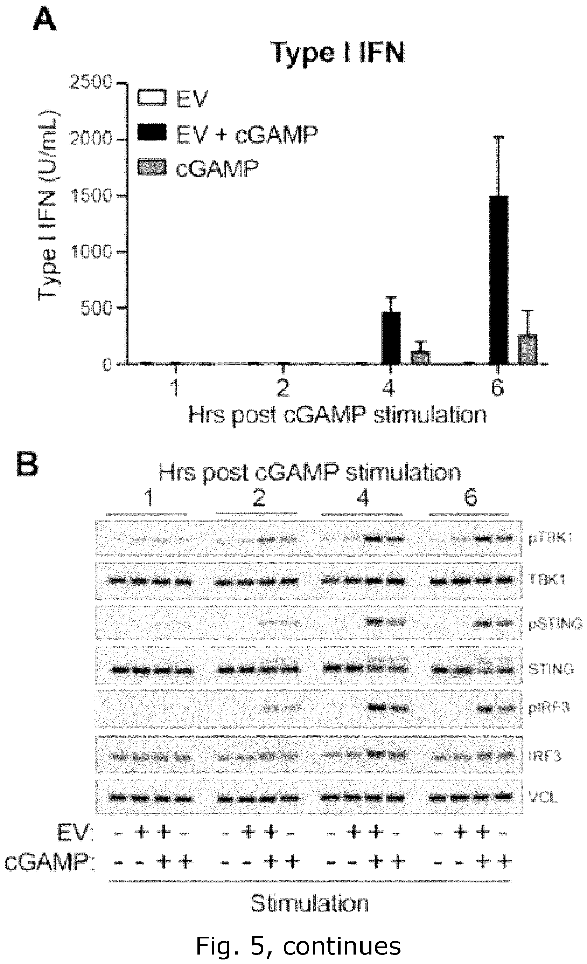

MT-EV pre-treatment of the cells resulted in increased Type I IFN production already after 4 hours of cGAMP stimulation supporting an early synergistic priming of the THP-1 cells by the T-EVs (Fig 5A). Corresponding Western blotting data showed potent signals for phosphorylated STING, TBK1, and IRF3 upon T-EV and cGAMP co-stimulation compared to cGAMP stimulation alone (Fig. 5B).

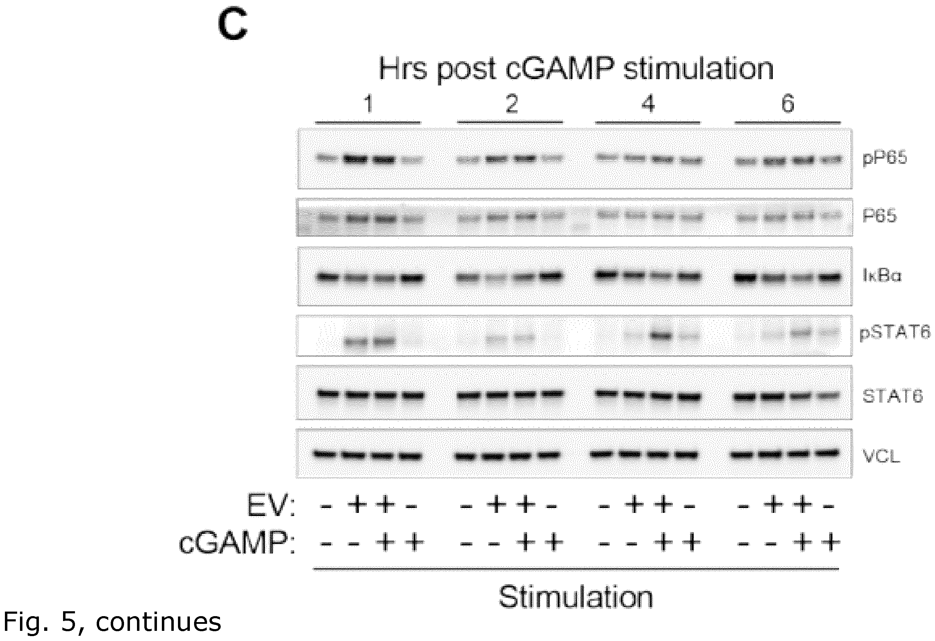

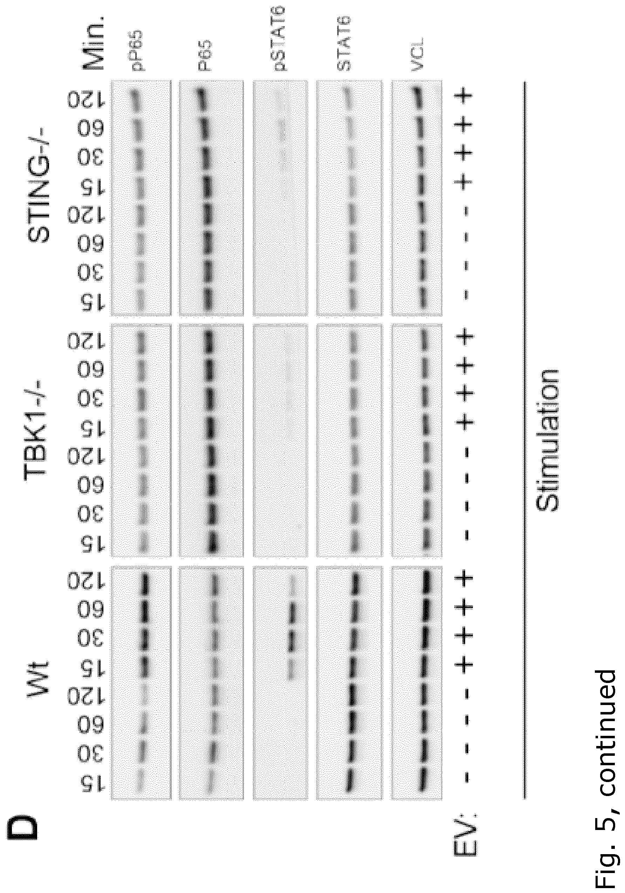

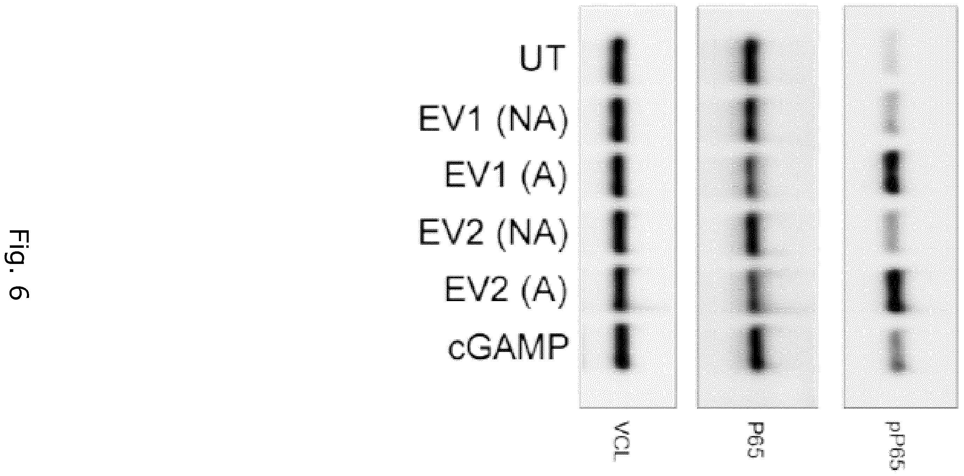

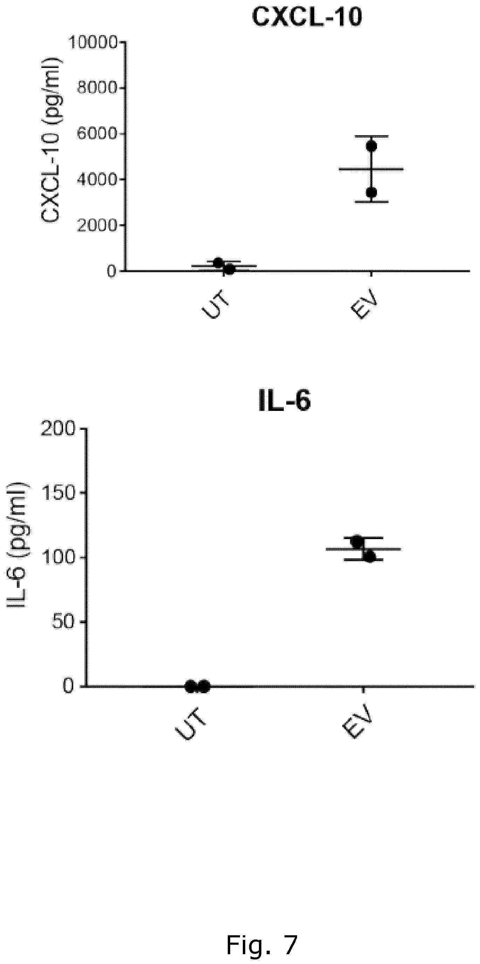

Activation of the STING-pathway has shown not only to signal through TBK1-IRF3, but also engage in the IKK-NF-kB as well as TBK1/IKK-STAT6 signalling pathway. Therefore, we next examined the activation of p65, a component of the NF-kB complex, and STAT6 following T-EVs and cGAMP stimulation. Interestingly, we found increased phosphorylation of both p65 and STAT6 upon co-stimulation with T-EVs and cGAMP compared to cGAMP stimulation alone (Fig. 7C). This further supports that the increased Type I IFN secretion is a result of enhanced activation of multiple STING-mediated signalling pathways. Interestingly, we did observe that stimulation of THP-1 cells with T-EVs alone resulted in early phosphorylation of both STAT6 and p65 (Fig. 5C) suggesting some level of discrepancy between the various STING-mediated signalling pathways. In THP-1 cells lacking TBK1 and STING, respectively, we showed that the phosphorylation of p65 and STAT6 was dependent on both TBK1 and STING (Fig. 5D). Furthermore, we showed that phosphorylation of p65 was only induced by T-EVs from activated CD4+ T cells (Fig. 6) consistent with our previous observation that primarily T-EVs from activated CD4+ T cells was capable of enhancing STING-induced Type I IFN secretion.

### Conclusion

Importantly, stimulation with T-EVs alone did not induce any expression of phosphorylated forms of STING, TBK1, or IRF3, indicating that T-EVs did not induce the pathway in absence of STING agonists.

### Example 6 - T-EVs alone induce secretion of pro-inflammatory cytokines from macrophages

### Aim of study

To examine the effect of T-EVs alone.

### Materials and methods

See example 1.

### Results

Although T-EV stimulation itself did not induce Type-I IFN secretion, we examined whether T-EV stimulation resulted in secretion of other pro-inflammatory cytokines known to be induced by the NF-kB signalling pathway. Notable, stimulation of human monocyte-derived macrophages with T-EVs alone resulted in secretion of both CXCL-10 and IL-6 (Fig. 7).

### Conclusion

Stimulation of human monocyte-derived macrophages with T-EV alone resulted in secretion of both CXCL-10 and IL-6.

### Example 7 - T-EVs transfer pro-inflammatory cytokines to enhance macrophage function

### Aim of study

Pro-inflammatory cytokines are among some of the molecules that induce NF-kB activation and cytokines. Therefore, we next analyzed the presence of inflammatory-associated cytokines in the T-EVs using multiplex immunoassay.

### Materials and methods

See example 1.

### Results

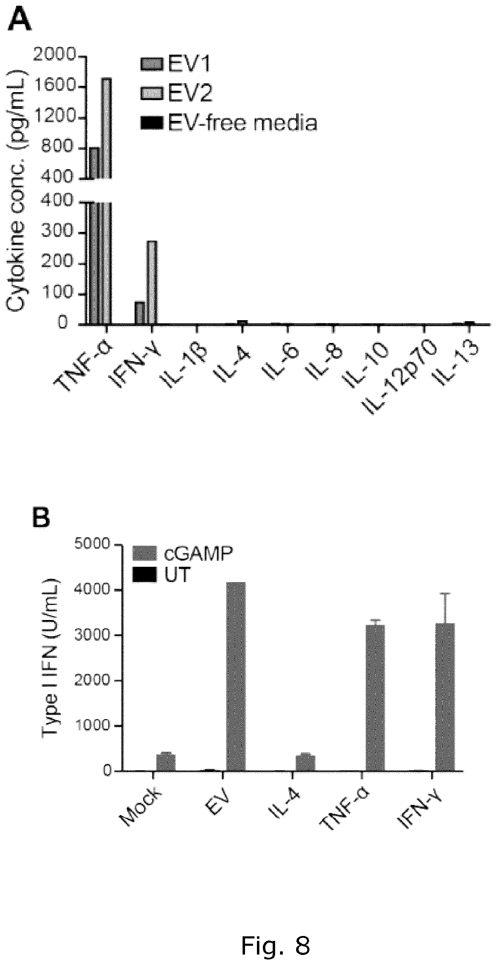

Among the inflammatory-associated cytokines we found that T-EVs contained both TNFa and IFNg at high levels (Fig. 8A). Next, we pre-treated THP-1 cells with recombinant TNFa or IFNg in similar amounts as was detected in the immunoassay, and found that both cytokines enhanced a STING-induced Type-I IFN production triggered by cGAMP, however neither of these cytokines alone induced Type-I IFN (Fig. 8B).

### Conclusion

These data suggest that the mode of action of T-EVs may in part involve transfer of pro-inflammatory cytokines capable of activating macrophages.

### Example 8 - Murine T-EVs enhance STING activation and induce NF-kB phosphorylation

### Aim of study

To investigate cross-species activities, it was explored whether T-EVs derived from activated murine CD4+ T cells functioned in a similar manner to what had been seen for human T-EVs.

### Materials and methods

See example 1.

### Results

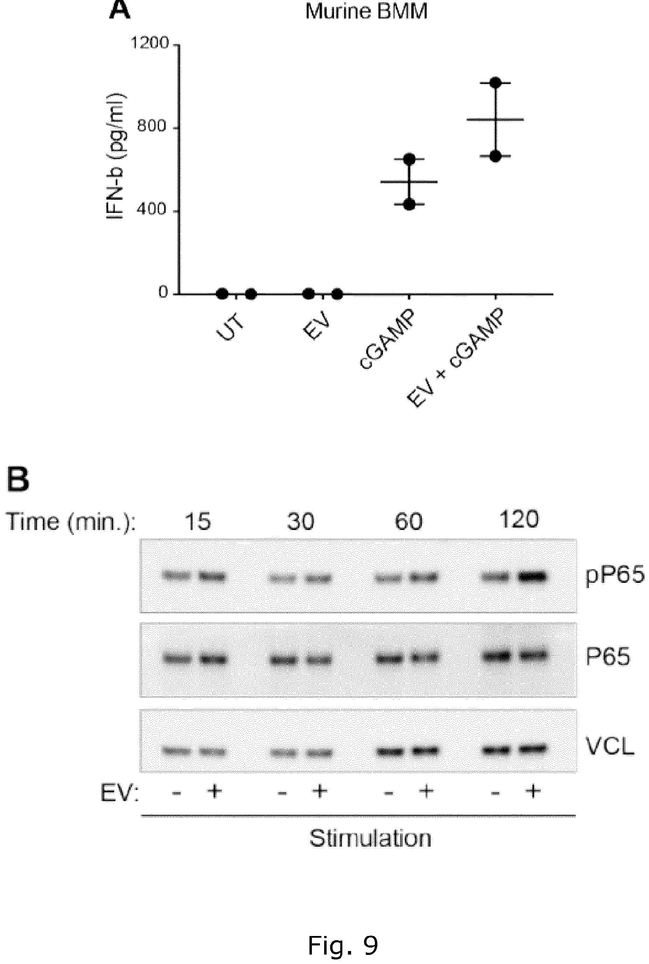

In order to investigate cross-species activity, murine bone-marrow derived macrophages (BMMs) were pre-treated with murine T-EVs and stimulated with cGAMP. Similar to the human setting, it was observed that murine T-EVs increased the STING-induced production of IFN-beta (Fig. 9A) and that stimulation of BMM with murine T-EVs alone induced phosphorylation of p65 (Fig. 9B).

### Conclusion

The data supports similar functionality of T-EVs from both human and mice.

### Example 9 - T-EVs potentiate the effect of cGAMP in reducing tumor growth in vivo

### Aim of study

To investigate whether murine T-EVs enhanced the effect of cGAMP in vivo.

### Materials and methods

See example 1.

### Results

In order to investigate whether murine T-EVs enhanced the effect of cGAMP in vivo, C57BL/6 mice bearing a subcutaneous MC38 colon adenocarcinoma were treated with either a low dose of cGAMP alone or EVs together with cGAMP. The mice receiving cGAMP alone showed a delayed tumor growth whereas mice receiving a combination of EVs and cGAMP showed complete tumor regression in 3 out of 4 mice (Fig. 10A, B).

### Conclusion

These data surprisingly indicate that (activated) CD4⁺ T-cell derived EVs prime the tumor microenvironment to increase the responsiveness to cGAMP stimulation and result in the activation of a more potent immune response supporting remission of the tumor.

### Example 10 - Murine T-EVs contain surface associated DNA capable of inducing type I IFN

### Aim of study

To determine the effect of surface associated DNA.

### Materials and methods

See example 1.

### Results

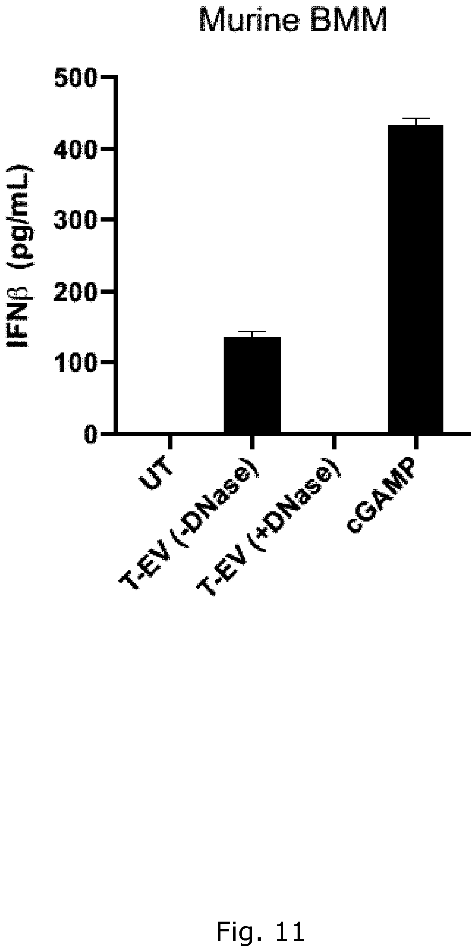

In all the experiments described above, T-EVs stripped for surface associated DNA were used to investigate the DNA-independent function of T-EVs. However, it has been shown that EVs can carry surface associated DNA capable of activating STING. To verify whether this is also the case for the T-EVs of the present invention, murine BMMs were stimulated with T-EVs that we either left untreated or treated with DNase I for 1 hr to remove surface associated DNA prior to stimulation. It was confirmed that T-EVs not treated with DNase induced secretion of IFN-beta whereas this effect was completely abolished upon treating the T-EVs with DNase I (Fig. 11).

### Conclusion

The data demonstrates the capability of T-EVs to carry surface associated DNA that can directly activate STING. This further supports that EVs from activated T cells can deliver both STING ligand (e.g. DNA) and priming compounds.

### References

1. Ghaffari A, Peterson N, Khalaj K, Vitkin N, Robinson A, Francis J-A, et al. STING agonist therapy in combination with PD-1 immune checkpoint blockade enhances response to carboplatin chemotherapy in high-grade serous ovarian cancer. Br J Cancer. 2018;119: 440-449.
2. Ager CR, Reilley MJ, Nicholas C, Bartkowiak T, Jaiswal AR, Curran MA. Intratumoral STING Activation with T-cell Checkpoint Modulation Generates Systemic Antitumor Immunity. Cancer Immunol Res. 2017;5: 676-684.
3. Moore E, Clavijo PE, Davis R, Cash H, Van Waes C, Kim Y, et al. Established T Cell-Inflamed Tumors Rejected after Adaptive Resistance Was Reversed by Combination STING Activation and PD-1 Pathway Blockade. Cancer Immunology Research. 2016. pp. 1061-1071.
4. Fu J, Kanne DB, Leong M, Glickman LH, McWhirter SM, Lemmens E, et al. STING agonist formulated cancer vaccines can cure established tumors resistant to PD-1 blockade. Sci Transl Med. 2015;7: 283ra52.
5. Deng L, Liang H, Xu M, Yang X, Burnette B, Arina A, et al. STING-Dependent Cytosolic DNA Sensing Promotes Radiation-Induced Type I Interferon-Dependent Antitumor Immunity in Immunogenic Tumors. Immunity. 2014;41: 843-852.
6. Francica BJ, Ghasemzadeh A, Desbien AL, Theodros D, Sivick KE, Reiner GL, et al. TNFa and Radioresistant Stromal Cells Are Essential for Therapeutic Efficacy of Cyclic Dinucleotide STING Agonists in Nonimmunogenic Tumors. Cancer Immunol Res. 2018;6: 422-433.
7. Torralba D, Baixauli F, Villarroya-Beltri C, Fernández-Delgado I, Latorre-Pellicer A, Acín-Pérez R, et al. Priming of dendritic cells by DNA-containing extracellular vesicles from activated T cells through antigen-driven contacts. Nat Commun. 2018;9: 2658.
8. Kitai Y, Kawasaki T, Sueyoshi T, Kobiyama K, Ishii KJ, Zou J, et al. DNA-Containing Exosomes Derived from Cancer Cells Treated with Topotecan Activate a STING-Dependent Pathway and Reinforce Antitumor Immunity. J Immunol. 2017;198: 1649-1659.
9. Mittelbrunn M, Gutiérrez-Vázquez C, Villarroya-Beltri C, González S, Sánchez-Cabo F, González MÁ, et al. Unidirectional transfer of microRNA-loaded exosomes from T cells to antigen-presenting cells. Nat Commun. 2011;2: 282.

## Claims

1. A composition comprising
- extracellular vesicles derived from activated T cells; and
- a STING stimulatory agent.

2. The composition according to claim 1, wherein the STING stimulatory agent is not derived from the T-cell from which the extracellular vesicles are derived, such as being exogenously delivered cyclic-di-nucleotides (CDNs) or DNA, preferably exogenously delivered CDNs or DNAse protected DNA.

3. The composition according to claim 1 or 2, wherein the STING stimulatory agent is selected from the group consisting of
• nucleic acids, such as cyclic-di-nucleotide (CDNs), such as cyclic 2'3' GMP-AMP (cGAMP), such as chemical stabilized forms of 2'3' cGAMP;
• enzymatic resistant double stranded DNA supporting intracellular cGAS activity;
• DNA damage repair (DDR) inhibitors including but not limited to: PARP inhibitors; ATM inhibitors, Topoisomerase inhibitors, DNA crosslinking agents, microtubule-targeting drugs, or antimetabolites;
• Small molecules capable of binding to and activating STING;
• Checkpoint inhibitors; and/or
• ENPP1 inhibitors.

4. The composition according to any of the preceding claims, wherein the isolated extracellular vesicles derived from activated T cells are obtained/obtainable by a process comprising
a) activating a composition comprising purified T cells using anti-CD3, anti-CD28 and IL-2; and
b) isolating activated extracellular vesicles derived from the activated T cells.

5. The composition according to any of the preceding claims, wherein said T cells are CD4⁺ T-cells and/or CD8+ T cells.

6. The composition according to any of the preceding claims 4-5, wherein the process further comprises a step c) of loading and/or coating the isolated extracellular vesicles with the STING stimulatory agent, such as cyclic-di-nucleotide (CDNs).

7. The composition according to claim 6, wherein DNA is loaded or coated, such as protected DNA, such as DNAse protected DNA.

8. The composition according to any of claims 4-7, wherein in step a) said purified T cells are from a subject in need of treatment or amelioration with the provided isolated extracellular vesicles derived from the activated T cells.

9. A kit or combination comprising
- extracellular vesicles derived from activated T cells; and
- a STING stimulatory agent.

10. The kit or combination according to claim 9, wherein the extracellular vesicles, derived from activated T cells, are isolated extracellular vesicles according to any of claims 3-7.

11. The composition according to any of claims 1-8 or kit or combination according to any of claims 9-10, for use as a medicament.

12. The composition according to any of claims 1-8 or kit or combination according to any of claims 8-10, for use in the treatment or amelioration of cancer.

13. The composition according to any of claims 1-8 or kit or combination according to any of claims 9-10 for use according to claim 12, wherein the composition is for treatment or amelioration of a subject who has undergone cancer therapy or is undergoing cancer therapy, or who is scheduled for cancer therapy of said cancer with a different anti-cancer therapy, such as with chemotherapeutics or by radiation.

14. Extracellular vesicles derived from activated T cells, for use in the treatment or amelioration of a subject suffering from cancer,
wherein said subject has undergone cancer therapy or is undergoing cancer therapy, or who is scheduled for cancer therapy, with a different anti-cancer therapy, such as with chemotherapeutics or by radiation.

15. The composition, or kit or combination according to claim 13 for use according to claim 13, or the extracellular vesicles derived from activated T cells according to claim 14 for use according to claim 14, wherein said therapy is selected from the group consisting of radiation therapy or therapy by chemotherapeutics.
